# EUROPEAN PATENT APPLICATION

(11) **EP 4 793 027 A1**
(43) Date of publication of application: **19.08.2026**
(21) Application number: 25219552.4
(22) Date of filing: 28.11.2025
(51) Int. Cl.: B29C 70/38, B32B 5/24, B64F 5/60, G01B 11/14, G01B 21/04, G01N 33/00

(54) **METHODS OF SELECTING MEASUREMENT EQUIPMENT**

(30) Priority: 14.02.2025 US 202519054161
(71) Applicant: The Boeing Company, Arlington, VA 22202 (US)
(72) Inventor: JONES, Stephen Paul, Arlington, 22202 (US); STEPHENS, Shaun Michael, Arlington, 22202 (US); BRADY, Steven K., Arlington, 22202 (US); SANDERS, Chanda D., Arlington, 22202 (US); DIEPENBROCK, Christopher David, Arlington, 22202 (US)
(74) Representative: Boult Wade Tennant LLP

(57) **Abstract**

Methods of selecting and qualifying measurement equipment are presented. A layup of adjacent strips of material is repeatedly measured along a material interface by measurement equipment to form measurement data. A deadband of gaps and overlaps is identified for the measurement equipment within the measurement data.

## Description

### FIELD

The present disclosure relates generally to composite materials and more specifically to measurement of gaps and overlaps in composite layups.

### BACKGROUND

When laminating broad goods or tapes, the presence and size of gaps and overlaps between adjacent strips of laminated material are often key parameters which are measured to verify an acceptable product was produced. Accurate measurement of gaps and overlaps is also valuable to understand the lamination process and make improvements to it. There are lower limits to the sizes of gaps and overlaps an automated measurement system can detect and measure.

Therefore, it would be desirable to have a method and apparatus that takes into account at least some of the issues discussed above, as well as other possible issues.

### SUMMARY

There is described herein a method of selecting measurement equipment. A layup of adjacent strips of material is repeatedly measured along a material interface by measurement equipment to form measurement data. A deadband of gaps and overlaps for the measurement equipment within the measurement data is identified.

There is described herein a method of selecting measurement equipment. A layup of adjacent strips of material is repeatedly measured along a material interface by measurement equipment to form measurement data, the material interface comprising a varying overlap and a varying gap forming a taper interface. A minimum overlap measurable to a desired reliability by the measurement equipment is identified from the measurement data. A minimum gap measurable to the desired reliability by the measurement equipment is identified from the measurement data. A deadband is defined for the measurement equipment between the minimum overlap and the minimum gap.

There is described herein a method of selecting measurement equipment. A second strip of material is laid up adjacent a first strip of material to form a taper interface with a varying gap and a varying overlap. The taper interface is repeatedly measured by measurement equipment to form measurement data. A deadband for the measurement equipment is identified between a minimum overlap measurable to a desired reliability from the measurement data and a minimum gap measurable to the desired reliability from the measurement data. The measurement equipment is selected for inspection of the composite part if the deadband of the measurement equipment is contained within a tolerance range for a composite part.

The features and functions can be achieved independently in various examples of the present disclosure or may be combined in yet other examples in which further details can be seen with reference to the following description and drawings.

### BRIEF DESCRIPTION OF THE DRAWINGS

The novel features believed characteristic of the illustrative examples are set forth in the appended claims. The illustrative examples, however, as well as a preferred mode of use, further objectives and features thereof, will best be understood by reference to the following detailed description of illustrative examples of the present disclosure when read in conjunction with the accompanying drawings, wherein:
Figure 1 is an illustration of an aircraft;
Figure 2 is an illustration of a block diagram of a manufacturing environment;
Figure 3 is an illustration of a layup of a composite material with a taper interface;
Figure 4 is an illustration of a plot of measured values and true values for a taper interface;
Figures 5A and 5b is a flowchart of a method of selecting measurement equipment;
Figures 6A and 6B is a flowchart of a method of selecting measurement equipment;
Figure 7 is a flowchart of a method of selecting measurement equipment;
Figure 8 is an illustration of an aircraft manufacturing and service method in a form of a block diagram; and
Figure 9 is an illustration of an aircraft in a form of a block diagram in which an illustrative example may be implemented.

### DETAILED DESCRIPTION

The illustrative examples recognize and take into account several considerations. The illustrative examples recognize and take into account that inspections including measurements of overlaps and gaps in a composite material can be used to verify the quality of composite laminations. The illustrative examples recognize and take into account that a measurement system analysis (MSA) of an automated measurement system is desirably performed before the measurement system is approved or "qualified" for use inspecting composite laminations. The illustrative examples recognize and take into account that it would be desirable to establish the smallest overlap and gap features a measurement system can accurately measure.

The illustrative examples recognize and take into account that in conventional evaluations of an automated measurement system that measures gaps and laps, multiple test laminates are laid up conventionally in parallel movements. The illustrative examples recognize and take into account that laps and gaps within the test laminates are manually measured. The illustrative examples recognize and take into account that laying up representative interfaces of most or all increments of overlaps and gaps may be almost prohibitively difficult or expensive. The illustrative examples recognize and take into account that in a traditional measurement system analysis using multiple interfaces to assess lap/gap measurement system performance, a good measure of how the automated measurement system performs with small laps and gaps may be very difficult to obtain.

The illustrative examples provide an efficient and accurate means to determine a measurement system's behavior in a small overlap-gap zone. The illustrative examples lay up strips of a composite material into a layup with a taper interface. The measurement equipment scans the taper interface between two strips. Machine measurements are analyzed to determine the measurement equipment's minimum reliably measurable overlap and gap values, which also establishes the measurement equipment's deadband (unreliable measurement zone). The illustrative examples provide an efficient and accurate means to determine a measurement equipment's behavior in this small lap-gap regime.

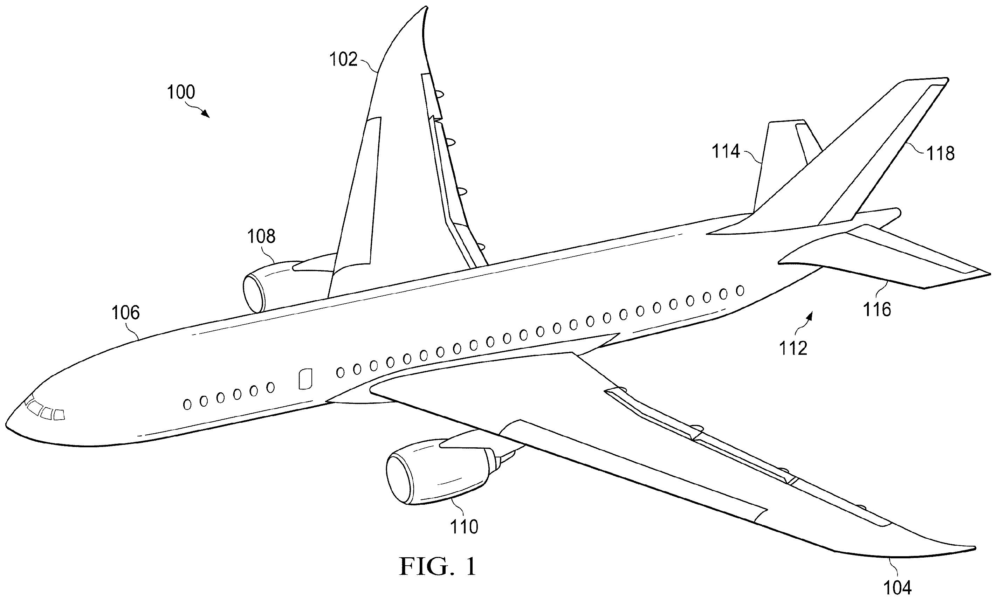

Turning now to Figure 1, an illustration of an aircraft is depicted in accordance with an illustrative example. Aircraft 100 has wing 102 and wing 104 attached to body 106. Aircraft 100 includes engine 108 attached to wing 102 and engine 110 attached to wing 104.

Body 106 has tail section 112. Horizontal stabilizer 114, horizontal stabilizer 116, and vertical stabilizer 118 are attached to tail section 112 of body 106.

Aircraft 100 is an example of an aircraft that can have composite parts inspected using measurement equipment selected using the illustrative examples. The illustrative examples provide a taper interface that can be used in qualifying measurement equipment for the inspection of composite parts during manufacture of aircraft 100. The illustrative examples can be used to qualify measurement equipment for manufacturing aircraft 100.

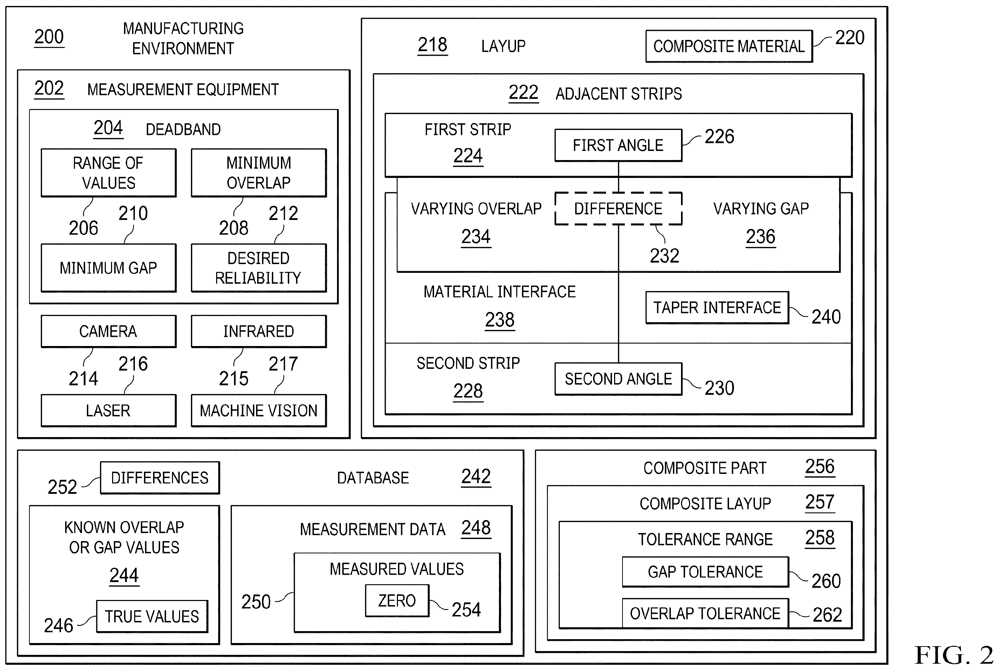

Turning now to Figure 2, an illustration of a block diagram of a manufacturing environment is depicted in accordance with an illustrative example. Manufacturing environment 200 can be used to manufacture portions of aircraft 100.

The illustrative examples provide a rapid test and method for evaluating the capabilities of measurement equipment 202. Measurement equipment 202 may also be referred to as material measurement equipment. Measurement equipment 202 can be an automated inspection/measurement system for inspecting composite layups. The illustrative examples provide a rapid test and methods for evaluating the capabilities of measurement equipment 202 in measuring small gaps and overlaps between adjacent lanes of laminated broad goods or tapes.

Measurement equipment 202 comprises any desirable type of measurement equipment configured to measure interfaces of composite layups. In some illustrative examples, measurement equipment 202 comprises one of camera 214, infrared 215, laser 216, or machine vision 217.

Measurement equipment 202 has lower limits to the sizes of gaps and overlaps measurement equipment 202 can detect and measure. In some illustrative examples, the sizes of gaps and overlaps that can be measured by measurement equipment 202 is influenced by the type of equipment. In some illustrative examples, the size of gaps and overlaps that can be measured by measurement equipment 202 is also additionally influenced by tolerances in manufacturing and mounting of measurement equipment 202.

The region including and between the thresholds of detectability of measurement equipment 202 is deadband 204 of measurement equipment 202. Determining the thresholds of detectability of measurement equipment 202 and determining the behavior of measurement equipment 202 when the feature to be measured (gap, lap, or abutment (i.e. "butt joint")) has a size between the thresholds is used to characterize measurement equipment 202 and qualify it for use. The threshold of detectability for measurement equipment 202 can be referred to as gap and lap thresholds. Minimum gap 210 is a gap threshold. Minimum overlap 208 is an overlap threshold. Deadband 204 comprises range of values 206 between and including minimum overlap 208 and minimum gap 210. When a feature's size is within deadband 204 of measurement equipment 202, the feature is desirably identified as zero 254.

Minimum overlap 208 is an overlap measurable to desired reliability 212 by measurement equipment 202. Minimum gap 210 is a gap measurable to desired reliability 212 by measurement equipment 202. Measurement equipment 202 measures with desired reliability 212 when the uncertainty associated with the measurements is small compared to tolerance range 258. The uncertainty associated with the measurement can be determined by repeated measurements of the same feature. In some illustrative examples, if the uncertainty of measurements of measurement equipment 202 is no more than 10% of tolerance range 258, the measurements can be considered very good. In some illustrative examples, if the uncertainty of measurements of measurement equipment 202 is no more than 30% of tolerance range 258, the measurements could be considered acceptable.

At least some illustrative examples provide a test to determine what measurement equipment 202 records while operating in deadband 204 as well as where deadband 204 begins and ends in terms of overlaps and gaps. Typically, it is desirable that measurement equipment 202 reports zero 254 when observing laps or gaps within deadband 204. The illustrative examples can be used to analyze the fitness of measurement equipment 202 for inspecting a laminated product, such as composite part 256.

Typically, small gaps and small overlaps are the measurements that fall into a deadband of a measurement system, such as deadband 204 of measurement equipment 202. It is desirable for high-quality composite layups to achieve a layup having almost no gaps or overlaps. Much of the population data for laid-up plies may regularly fall inside the deadband of a piece of measurement equipment, such as measurement equipment 202. The knowledge of the behavior of measurement equipment 202 when attempting to measure gaps and overlaps within deadband 204 is valuable. The behavior of measurement equipment 202 when measuring overlaps and gaps within deadband 204 is important to understanding its ability to accurately construct a lap/gap population which represents the true distribution of laps and gaps in a given laminate. Characterizing deadband 204 of measurement equipment 202 can include identifying when features with sizes within deadband 204 of measurement equipment 202 produce measurements that are not zero 254. If measurement equipment 202 produces non-zero measurements for features within deadband 204 at an undesirable rate, measurement equipment 202 may not be selected for measurement of composite part 256.

Composite layup 257 is a portion of composite part 256. Composite layup 257 is formed of composite material. Gap tolerance 260 and overlap tolerance 262 are set based on a type of the composite material, the intended use for composite part 256, the position of composite layup 257 within composite part 256 and other factors. Typically, desired values of the true population of overlaps and gaps in a commercial composite layup, such as composite layup 257, are set for a desired quality of the composite product, such as composite part 256. In this and certain other illustrative examples, composite layup 257 for composite part 256 have gap tolerance 260 and overlap tolerance 262 for tolerance range 258. Other composite layups in composite part 256 may have a different tolerance range based on quality or material considerations.

Measurement equipment 202 is used to aid in determination of whether or not a composite laminate, such as composite part 256, has met the requirements. Behavior of measurement equipment 202 when measuring laps and gaps of all sizes relevant to the layup determines whether measurement equipment 202 is suitable to use for product acceptance. Layup 218 helps to elucidate the extent of deadband 204 and the behavior of measurement equipment 202 when laps and gaps fall within deadband 204.

Deadband 204 is compared to a tolerance range 258 for composite layup 257 to determine if measurement equipment 202 is capable of inspecting composite layup 257 for a desired quality of composite part 256. If deadband 204 is too wide for tolerance range 258 of composite layup 257, measurement equipment 202 may not be suitable for use as a product-acceptance tool for composite part 256. Understanding deadband 204 of measurement equipment 202 is important to identifying nonconforming product and removing composite parts with undesirable quality from production.

Measurement equipment 202 could report an erroneously-large population of overlaps and gaps due to poor performance in deadband 204. An erroneously large population of overlaps and gaps could cause unnecessary scrap of a conforming part. Understanding deadband 204 of measurement equipment 202 is important to guarding against unnecessary scrap and the associated costs.

Measurement equipment 202 is evaluated using layup 218 with taper interface 240. An evaluation using layup 218 having taper interface 240 can be referred to as a taper test. If the taper test reveals an undesirable deadband 204 for measurement equipment 202, such as deadband 204 being very large or having noisy data when true laps and gaps fall within deadband 204, measurement equipment 202 may only be appropriate for select composite parts. Deadband 204 could indicate that measurement equipment 202 is suitable for product acceptance of some composite parts, but not others.

Composite layup 257 has tolerance range 258. If tolerance range 258 has very tight lap/gap requirements, for example having overlap tolerance 262 and gap tolerance 260 of a size near zero or substantially near zero, measurement systems for acceptance of composite layup 257 have a small deadband. When tolerance range 258 has tight requirements, composite layup 257 should not be inspected for product acceptance or rejection by measurement equipment with deadband behavior inconsistent with the level of tolerances in tolerance range 258. Measurement equipment 202 can have deadband 204 measured by a taper test utilizing layup 218. If deadband 204 is very wide, very many of the overlaps and gaps of composite layup 257 may fall within deadband 204 of measurement equipment 202. If many of the overlaps and gaps of composite layup 257 fall within deadband 204, little to no knowledge of the true lap/gap state of composite layup 257 will be known with which to make a determination about the fitness of composite layup 257 for service. If measurement equipment 202 is determined, using a taper test utilizing layup 218, to routinely report erroneous measured values for overlaps and gaps which fall within deadband 204, this also is undesirable for determining quality of composite part 256. If measurement equipment 202 produces an undesirably skewed representation of the true population of laps and gaps, composite parts may be erroneously scrapped when the composite parts are actually within tolerance.

Even if deadband 204 is undesirably wide for one composite part with tight tolerances, measurement equipment 202 can still be suitable for determining the fitness of different composite laminates with looser lap/gap tolerances. Measurement equipment 202 can be suited for measurement and evaluation of composite layup 257 when tolerance range 258 is larger than deadband 204. The larger the difference between tolerance range 258 and deadband 204, the more certainty that measurement equipment 202 is appropriate to measure composite layup 257. Size of deadband 204 can qualify measurement equipment 202 for some composite layups with wider tolerance ranges and disqualify measurement equipment 202 for composite layups with tolerance ranges within or mostly within deadband 204. If the likely true population of gaps and overlaps for composite layup 257 is much broader in size than the extent of deadband 204, measurement equipment 202 may be used for measurement of composite layup 257.

Determining the thresholds of detectability of measurement equipment 202 is performed using layup 218. Layup 218 comprises composite material 220. Composite material 220 takes the form of any desirable type of pre-impregnated or dry fiber composite. In some illustrative examples, adjacent strips 222 of material comprise at least one of a thermoplastic prepreg material, a thermoset prepreg material, or a dry fiber material. An example of composite material 220 can be unidirectional carbon fiber tape pre-impregnated with an epoxy resin, commonly used in manufacturing aerospace components such as composite wing skins. Such tape is sometimes known as carbon fiber reinforced polymer (CFRP) tape.

Forming layup 218 comprises laying up first strip 224 and second strip 228 of composite material 220. To form layup 218, second strip 228 is laid up at a shallow angle relative to first strip 224. In this and certain other illustrative examples, first strip 224 is laid up at a first angle 226 and second strip 228 is laid up at second angle 230. Difference 232 between first angle 226 and second angle 230 causes material interface 238 between first strip 224 and second strip 228 to take the form of taper interface 240. Difference 232 can have any desirable value. In some illustrative examples, difference 232 is up to two degrees. In some illustrative examples, difference 232 is one degree or less. In some illustrative examples, difference 232 is below one degree. In some illustrative examples, difference 232 is less than 0.5 degree. In some illustrative examples, difference 232 is 0.11 degree or about 0.11 degree.

Material interface 238 is the interface between edges of first strip 224 and second strip 228. In this and certain other illustrative examples, material interface 238 takes the form of taper interface 240. Taper interface 240 comprises varying overlap 234 and varying gap 236. Varying overlap 234 transitions to varying gap 236 at a point of no overlap or gap.

In some illustrative examples, second strip 228 can be laid up relative to first strip 224 so second strip 228 starts in an overlap condition and ends in a gap condition. In other illustrative examples, second strip 228 can be laid up relative to first strip 224 so second strip 228 starts in a gap condition and ends in an overlap condition.

Although layup 218 is depicted as comprising first strip 224 and second strip 228, in some illustrative examples, first strip 224 is one of a plurality of adjacent strips laid up at first angle 226. Additionally, although layup 218 is depicted as comprising second strip 228, in some illustrative examples, second strip 228 is one of a plurality of adjacent strips laid up at second angle 230. In these and ceertain other illustrative examples, a set of multiple adjacent second strips are laid up at a shallow angle to the first set of adjacent strips.

In some illustrative examples, the entire layup described above can be called a "taper interface". True overlap and gap values 244 are accurately and precisely measured periodically along the length of taper interface 240 using a device such as a calibrated microscope.

Measurement equipment 202 is used to repeatedly measure taper interface 240 along its length, typically from lap-to-gap, and then gapto-lap. In these and certain other illustrative examples, measurement equipment 202 can move from varying overlap 234 towards varying gap 236 and then from varying gap 236 to varying overlap 234.

Measurement data 248 from measurement equipment 202 repeatedly measuring taper interface 240 is evaluated to quantify deadband 204. Measurement data 248 from measurement equipment 202 repeatedly measuring taper interface 240 is evaluated to determine performance in and near deadband 204.

Differences 252 between measured values 250 of measurement data 248 and known overlap or gap values 244 are used to determine deadband 204. Known overlap or gap values 244 can also be referred to as true values 246. True values 246 for taper interface 240 can be known by accurately and precisely measuring its overlap or gap values periodically along its length using a device such as a calibrated microscope.

In some illustrative examples, of measurement data 248 and known overlap or gap values 244 can be saved in database 242. In other illustrative examples, measurement data 248 and known overlap or gap values 244 can be saved in separate locations.

Layup 218 can be laminated by hand or by automated lamination equipment. Composite layup 257 can be laminated by hand or by automated lamination equipment. An example of such material is unidirectional carbon fiber tape pre-impregnated with an epoxy resin, commonly used in manufacturing aerospace components such as composite wing skins. Such tape is sometimes known as carbon fiber reinforced polymer (CFRP) tape.

The illustration of manufacturing environment 200 in Figure 2 is not meant to imply physical or architectural limitations to the manner in which an illustrative example may be implemented. Other components in addition to or in place of the ones illustrated may be used. Some components may be unnecessary. Also, the blocks are presented to illustrate some functional components. One or more of these blocks may be combined, divided, or combined and divided into different blocks when implemented in an illustrative example.

For example, in some illustrative examples, first strip 224 is one of a plurality of first strips. In these and certain other illustrative examples, the plurality of first strips can be laid up adjacently at a same angle during a layup step. In other illustrative examples, second strip 228 is one of a plurality of second strips. In these and certain other illustrative examples, the plurality of second strips can be laid up adjacently at the same second angle relative to first strip 224 or a plurality of first strips in a single layup step.

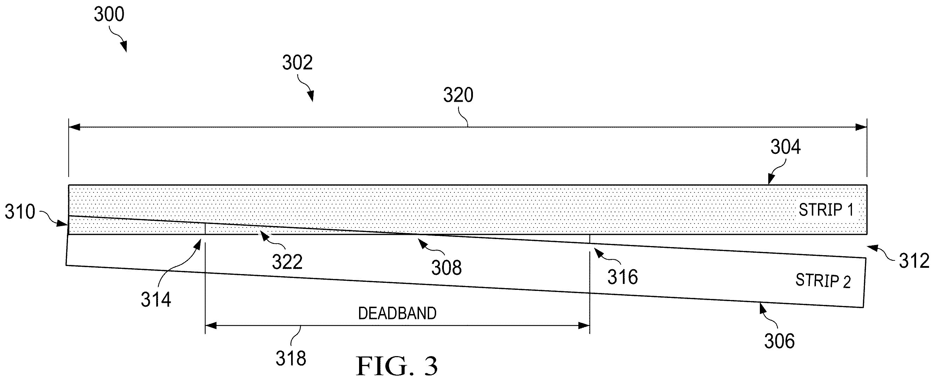

Turning now to Figure 3, an illustration of a layup of a composite material with a taper interface is depicted in accordance with an illustrative example. View 300 is a top view of layup 302. Layup 302 can be used to evaluate measurement systems to be used to inspect composite parts for aircraft 100 of Figure 1. Layup 302 can be a physical implementation of layup 218 of Figure 2.

Layup 302 comprises first strip 304 and second strip 306. At location 308, there is no gap or overlap between first strip 304 and second strip 306. Layup 302 comprises varying overlap 310 of second strip 306 atop first strip 304. In this and certain other illustrative examples, second strip 306 overlaps first strip 304 in varying overlap 310. Layup 302 comprises varying gap 312 between second strip 306 and first strip 304.

First strip 304 and then second strip 306 of tape is laid down roughly adjacent and parallel. In some illustrative examples, it can be easier to keep second strip 306 of tape going in a straight line when laying it up from an overlap condition, varying overlap 310, to a gap condition, varying gap 312.

Layup 302 provides an efficient and accurate means to determine a measurement equipment's (not depicted) behavior in this small overlap-gap zone. Layup 302 provides an efficient and accurate means to determine a measurement system's behavior for both gaps and overlaps.

To determine behavior of measurement equipment (not depicted), the measurement equipment scans interface 322 between first strip 304 and second strip 306. Machine measurements are analyzed to determine the measurement equipment's minimum reliably measurable overlap and gap values, which also establishes the equipment's deadband (unreliable measurement zone), deadband 318.

To form layup 302, one strip, first strip 304, is laminated onto a layup mandrel or other suitable lamination substrate. In some illustrative examples, for a narrow tape it can be desirable to laminate more than one strip together so that abutting, neighboring strips keep each other from "wandering" laterally to the layup direction. In these and certain other non-depicted examples, additional first strips can be laid up abutting each other. In some illustrative examples, multiple feet of material are laminated. Next, the layup direction is altered slightly so it is no longer exactly parallel with the original layup direction of first strip 304. Starting from the same or about the same position as first strip 304, second strip 306 is laminated for the same or about the same length as first strip 304. In some illustrative examples, multiple neighboring second strips are laid up abutting each other. In some illustrative examples, second strip 306 starts such that some of the new material overlaps the existing layup, first strip 304. Because of the angular offset of second strip 306, the overlap of the new material of second strip 306 on the old material of first strip 304 becomes less and less until, at some point, there is no overlap at location 308. As the layup of second strip 306 continues, there then forms an ever-widening gap between the new material of second strip 306 and the originally-laid-up material of first strip 304.

In some illustrative examples, both strips are laid up from left to right in Figure 3. The uppermost strip in Figure 3, first strip 304, was laid up first, and then the lower strip in view 300 of Figure 3, second strip 306, was laid up. In some illustrative examples, the lower strip, second strip 306, is initially partially overlapping the upper strip. Laying up second strip 306 or a plurality of second strips in the same or substantially the same direction as the layup of first strip 304 can be easier than laying up first strip 304 and second strip 306 in different directions. For automated layup of carbon fiber (CFRP) tape, it is typically easier to keep second strip 306 of tape going in a straight line when laying it up from an overlap condition to a gap condition rather than the opposite direction.

In view 300, varying overlap 310 is at the left side of view 300, and varying gap 312 is at the right side of view 300. In some illustrative examples, an angle between first strip 304 and second strip 306 can be 0.11 degrees or about 0.11 degrees. This may also be described as a difference in angles of layup for first strip 304 and second strip 306 is a difference of 0.11 degrees. In some illustrative examples, using an angle of 0.11 degrees or approximately 0.11 degrees can achieve a variation in the overlap (or gap) width of 0.002" per inch /0.002cm per centimetre of layup travel (or approximately 0.002" per inch/approximately 0.002cm per centimetre of layup travel). Although a variation of 0.002" per inch/0.002cm per centimetre corresponding to an angular difference of 0.11 degrees or about 0.11 degrees is discussed, but other angles can be utilized. The rate of change of the overlap (or gap) should be chosen to be relevant and useful for the measurement equipment being evaluated. The amount of initial overlap is chosen such that the measurement equipment can easily measure it, and the amount of final gap between the first strip and second strip of tape is also chosen such that it is easily measurable.

This overlap-and-gap layup, layup 302, sometimes called a "taper" layup, presents overlaps and gaps of continuously varying width for the measurement system to measure. Providing layup 302 provides advantages over attempting to make many discrete layups containing various discrete overlap or gap sizes. Layup 302 can be created quickly and provides all the lap and gap sizes for quantifying the measurement equipment's deadband. Measurement data obtained using layup 302 by the measurement equipment outside of its deadband is also useful in quantifying the measurement equipment's performance. Layup 302 can be used to determine the minimum overlap and gap sizes the measurement equipment can successfully measure, determine the measurement equipment's behavior in regions where the overlap or gap width is below the minimum measurable size, and determine the measurement equipment's behavior as it transitions from overlaps (or gaps) it can measure to overlaps (or gaps) it cannot.

Taper interface 322 can be repeatedly measured using measurement equipment, such as measurement equipment 202 of Figure 2, along length 320 of taper interface 322. Repeated measurements along length 320 of taper interface 322 can be used to determine the measurement equipment's behavior in its deadband.

Deadband 318 is depicted as an example of a deadband for an instance of measurement equipment. Minimum gap 316 is an example of a gap threshold for a piece of measurement equipment. Minimum overlap 314 is an example of an overlap threshold for a piece of measurement equipment. Deadband 318 comprises a range of values between and including minimum overlap 314 and minimum gap 316. Deadband 318 is only an example and is non-limiting. Each deadband will be unique to a piece of measurement equipment.

Some automated measurement equipment is of a scanning type, in which the measurement equipment moves along layup 302 and takes measurements as it moves. If the automated measurement system is of a scanning type, the measurement equipment can be used to take data for testing during layup of second strip 306 (or strips). In some illustrative examples, if the automated measurement system is of a scanning type, it can also be used to perform as many scans as desired from left to right in the orientation of Figure 3 following layup. When measuring in this direction, the system first measures overlaps, and then gaps. Following lamination, layup 302 can also be scanned from right to left in the orientation of Figure 3, in which case a scanning type measurement system first measures gaps and then overlaps.

Automated measurement equipment which can measure gaps or overlaps in more than one location at once can also be evaluated. Measurement equipment which can measure gaps or overlap in more than one location at once can also measure layup 302 as many times as are useful to determine its measurement behavior. Layup 302 is a rapid, efficient means to present a range of overlap and gap sizes to the measurement equipment independent of measurement equipment type.

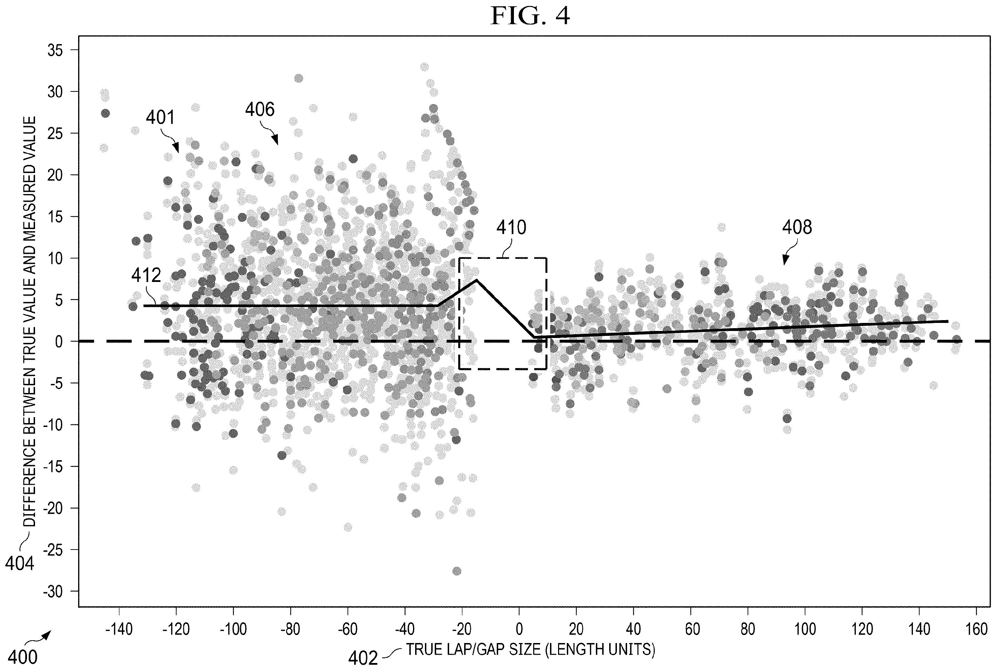

Turning now to Figure 4, an illustration of a plot of measured values and true values for a taper interface is depicted in accordance with an illustrative example. Graph 400 can be measurement data taken to qualify measurement equipment for inspection of composite parts of aircraft 100. Graph 400 can be generated from measurement data 248 and true values 246 of Figure 2. Graph 400 can be generated from measurement data generated from layup 302 of Figure 3.

Graph 400 depicts measurement error bias and variation. Graph 400 has x-axis 402 of the true feature sizes also known as true lap/gap size. Graph 400 has y-axis 404 of a difference between true values and measured values. Y-axis 404 can be described as bias in lap/gap measurements (measured values minus true values). Graph 400 comprises plurality of overlap data points 406 and plurality of gap data points 408.

As depicted, graph 400 contains a plurality of points for each value along x-axis 402. Measurement equipment repeatedly scans the taper interface of the layup to generate multiple measurement values for each true value on x-axis 402. A region between plurality of overlap data points 406 and plurality of gap data points 408 comprises a deadband. Measured values within the deadband are primarily zero value data points. Curve 412 illustrates the measurement bias behavior of measurement equipment across the range of relevant lap and gap sizes. The taper layup and methods of the illustrative examples allow for rapid quantification of the measurement behavior of measurement equipment in the region captured by box 410. The region captured by box 410 represents the deadband of the measurement equipment.

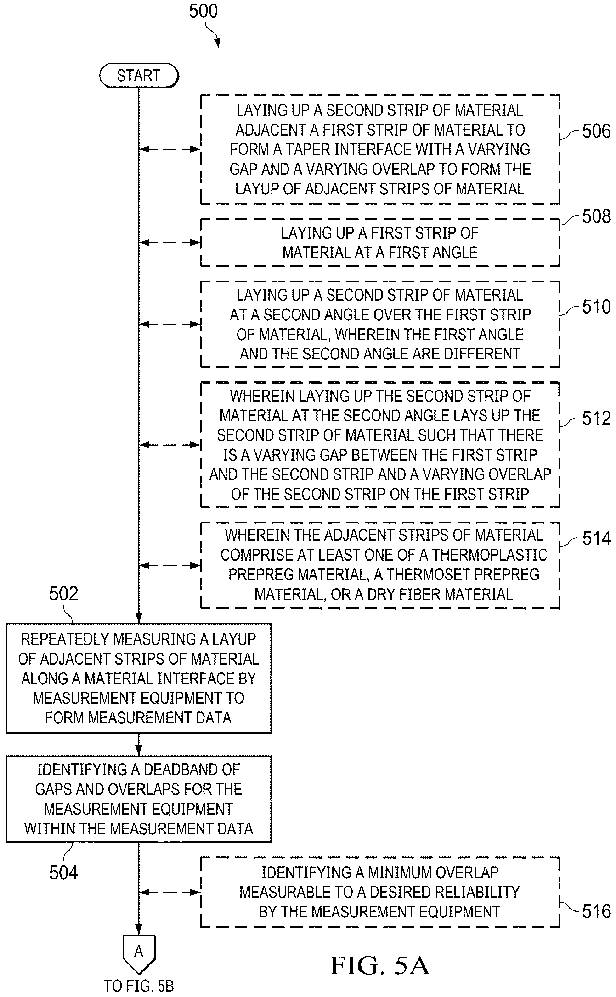

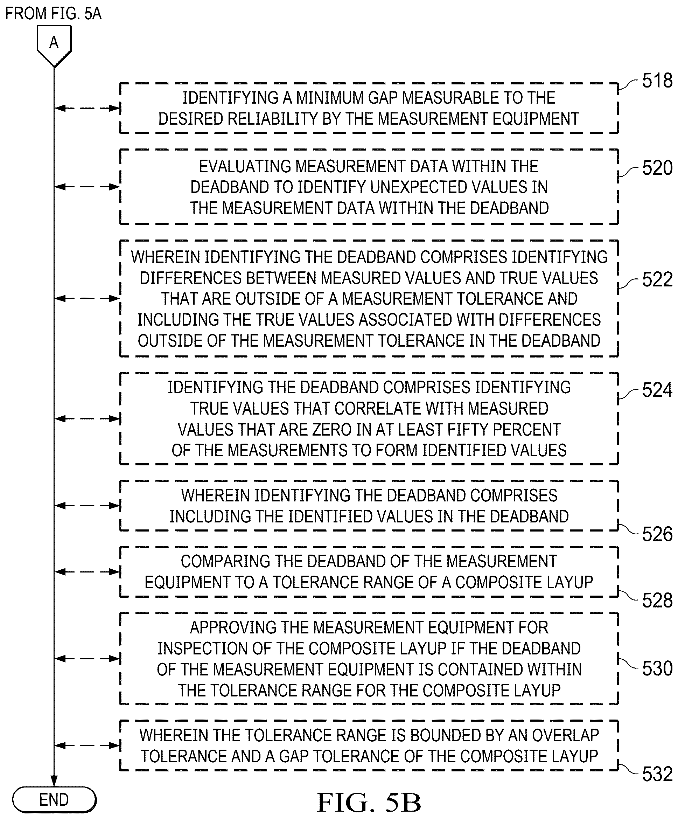

Turning now to Figures 5A and 5B, a flowchart of a method of selecting measurement equipment is depicted in accordance with an illustrative example. Method 500 can be used to select measurement equipment for inspecting a composite layup of a part for aircraft 100 of Figure 1. Method 500 can be used to identify deadband 204 of measurement equipment 202 of Figure 2. Method 500 can be performed using layup 302 of Figure 3. Method 500 can result in measurement data 401 of Figure 4.

Method 500 repeatedly measures a layup of adjacent strips of material along a material interface by measurement equipment to form measurement data (operation 502). Method 500 identifies a deadband of gaps and overlaps for the measurement equipment within the measurement data (operation 504). Afterwards, method 500 terminates.

In some illustrative examples, method 500 lays up a second strip of material adjacent a first strip of material to form a taper interface with a varying gap and a varying overlap to form the layup of adjacent strips of material (operation 506). The varying overlap and the varying gap are at a small enough variation to generate multiple measurements within a deadband. In some illustrative examples, the varying overlap and the varying gap are laid up at a change of 0.002" per inch or approximately 0.002" per inch (0.002cm per centimetre or approximately 0.002cm per centimetre) of layup travel.

In some illustrative examples, method 500 lays up a first strip of material at a first angle (operation 508). The first angle is relative to a layup tool or table.

In some illustrative examples, method 500 lays up a second strip of material at a second angle over the first strip of material, wherein the first angle and the second angle are different (operation 510). A difference between the first angle and the second angle is less than one degree. In some illustrative examples, a difference between the first angle and the second angle is less than 0.5 degrees. In some illustrative examples, a difference between the first angle and the second angle is 0.11 degrees or approximately 0.11 degrees.

In some illustrative examples, laying up the second strip of material at the second angle lays up the second strip of material such that there is a varying gap between the first strip and the second strip and a varying overlap of the second strip on the first strip (operation 512).

In some illustrative examples, the adjacent strips of material comprise at least one of a thermoplastic prepreg material, a thermoset prepreg material, or a dry fiber material (operation 514). In some illustrative examples, the adjacent strips of material are formed of a same material as a composite layup to be inspected by the measurement equipment.

In some illustrative examples, method 500 identifies a minimum overlap measurable to a desired reliability by the measurement equipment (operation 516). In some illustrative examples, method 500 identifies a minimum gap measurable to the desired reliability by the measurement equipment (operation 518).

In some illustrative examples, method 500 evaluates measurement data within the deadband to identify unexpected values in the measurement data within the deadband (operation 520). In some illustrative examples, the unexpected values are non-zero values.

In some illustrative examples, identifying the deadband comprises identifying differences between measured values and true values that are outside of a measurement tolerance and including the true values associated with differences outside of the measurement tolerance in the deadband (operation 522). In some illustrative examples, identifying the deadband comprises identifying true values that correlate with measured values that are zero in at least fifty percent of the measurements to form identified values (operation 524). In some illustrative examples, identifying the deadband comprises identifying true values that correlate with measured values that are zero in at least seventy percent of the measurements to form identified values. identifying the deadband comprises identifying true values that correlate with measured values that are zero in at least ninety percent of the measurements to form identified values. In some illustrative examples, identifying the deadband comprises including the identified values in the deadband (operation 526).

In some illustrative examples, method 500 compares the deadband of the measurement equipment to a tolerance range of a composite layup (operation 528). In some illustrative examples, method 500 approves the measurement equipment for inspection of the composite layup if the deadband of the measurement equipment is contained within the tolerance range for the composite layup (operation 530). In some illustrative examples, the tolerance range is bounded by an overlap tolerance and a gap tolerance of the composite layup (operation 532).

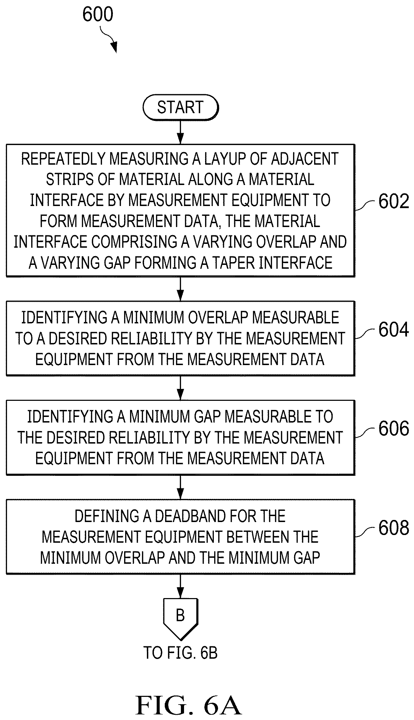

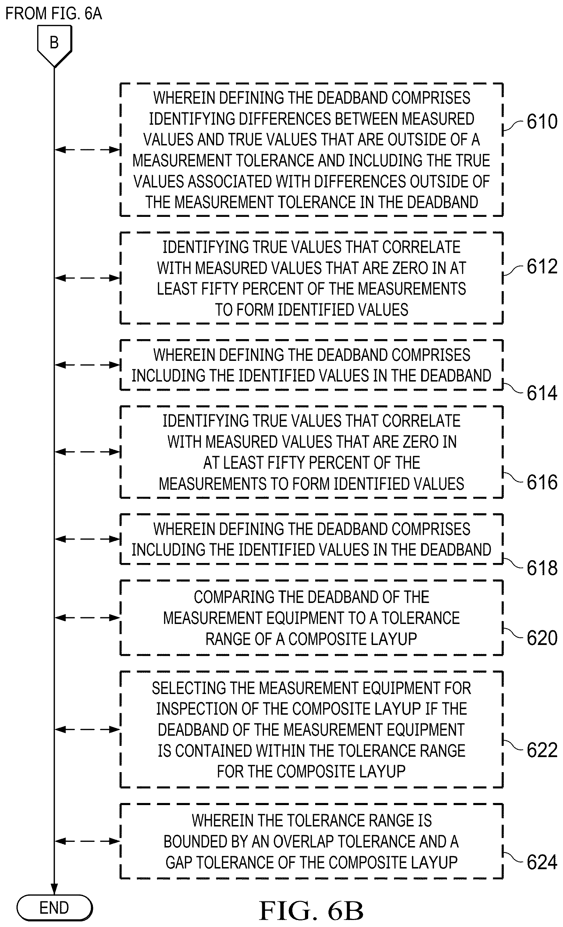

Turning now to Figures 6A and 6B, a flowchart of a method of selecting measurement equipment is depicted in accordance with an illustrative example. Method 600 can be used to select measurement equipment for inspecting a composite layup of a part for aircraft 100 of Figure 1. Method 600 can be used to identify deadband 204 of measurement equipment 202 of Figure 2. Method 600 can be performed using layup 302 of Figure 3. Method 600 can result in measurement data 401 of Figure 4.

Method 600 repeatedly measures a layup of adjacent strips of material along a material interface by measurement equipment to form measurement data, the material interface comprising a varying overlap and a varying gap forming a taper interface (operation 602). Method 600 identifies a minimum overlap measurable to a desired reliability by the measurement equipment from the measurement data (operation 604). Method 600 identifies a minimum gap measurable to the desired reliability by the measurement equipment from the measurement data (operation 606). Method 600 defines a deadband for the measurement equipment between the minimum overlap and the minimum gap (operation 608). Afterward, method 600 terminates.

In some illustrative examples, defining the deadband comprises identifying differences between measured values and true values that are outside of a measurement tolerance and including the true values associated with differences outside of the measurement tolerance in the deadband (operation 610). In some illustrative examples, method 600 identifies true values that correlate with measured values that are zero in at least fifty percent of the measurements to form identified values (operation 612). In some illustrative examples, defining the deadband comprises including the identified values in the deadband (operation 614).

In some illustrative examples, method 600 identifies true values that correlate with measured values that are zero in at least fifty percent of the measurements to form identified values (operation 616). In some illustrative examples, defining the deadband comprises including the identified values in the deadband (operation 618).

In some illustrative examples, method 600 compares the deadband of the measurement equipment to a tolerance range of a composite layup (operation 620). Method 600 selects the measurement equipment for inspection of the composite layup if the deadband of the measurement equipment is contained within the tolerance range for the composite layup (operation 622). In some illustrative examples, the tolerance range is bounded by an overlap tolerance and a gap tolerance of the composite layup (operation 624).

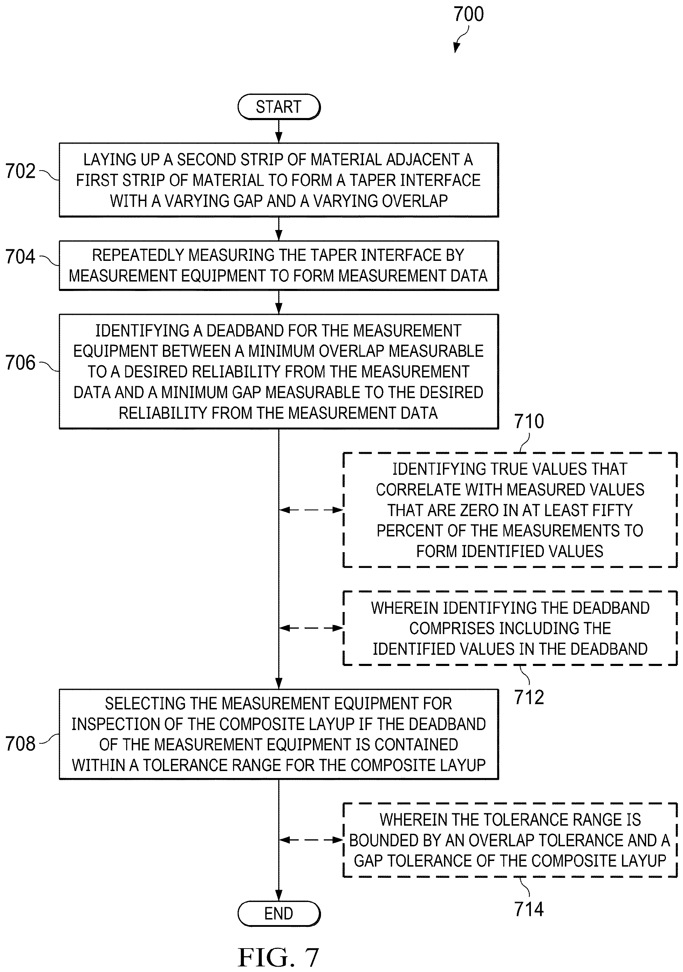

Turning now to Figure 7, a flowchart of a method of selecting measurement equipment is depicted in accordance with an illustrative example. Method 700 can be used to select measurement equipment for inspecting a composite layup of a part for aircraft 100 of Figure 1. Method 700 can be used to identify deadband 204 of measurement equipment 202 of Figure 2. Method 700 can be performed using layup 302 of Figure 3. Method 700 can result in measurement data 401 of Figure 4.

Method 700 lays up a second strip of material adjacent a first strip of material to form a taper interface with a varying gap and a varying overlap (operation 702). Method 700 repeatedly measures the taper interface by measurement equipment to form measurement data (operation 704). Method 700 identifies a deadband for the measurement equipment between a minimum overlap measurable to a desired reliability from the measurement data and a minimum gap measurable to the desired reliability from the measurement data (operation 706). Method 700 selects the measurement equipment for inspection of the composite layup if the deadband of the measurement equipment is contained within a tolerance range for a composite layup (operation 708). Afterwards, method 700 terminates.

In some illustrative examples, method 700 identifies true values that correlate with measured values that are zero in at least fifty percent of the measurements to form identified values (operation 710). In some illustrative examples, identifying the deadband comprises including the identified values in the deadband (operation 712). In some illustrative examples, the tolerance range is bounded by an overlap tolerance and a gap tolerance of the composite layup (operation 714).

As used herein, the phrase "at least one of," when used with a list of items, means different combinations of one or more of the listed items may be used and only one of each item in the list may be needed. For example, "at least one of item A, item B, or item C" may include, without limitation, item A, item A and item B, or item B. This example also may include item A, item B, and item C or item B and item C. Of course, any combinations of these items may be present. In other examples, "at least one of" may be, for example, without limitation, two of item A; one of item B; and ten of item C; four of item B and seven of item C; or other suitable combinations. The item may be a particular object, thing, or a category. In other words, at least one of means any combination items and number of items may be used from the list but not all of the items in the list are required.

As used herein, "a number of," when used with reference to items means one or more items.

The flowcharts and block diagrams in the different depicted examples illustrate the architecture, functionality, and operation of some possible implementations of apparatuses and methods in an illustrative example. In this regard, each block in the flowcharts or block diagrams may represent at least one of a module, a segment, a function, or a portion of an operation or step.

In some alternative implementations of an illustrative example, the function or functions noted in the blocks may occur out of the order noted in the figures. For example, in some cases, two blocks shown in succession may be executed concurrently or substantially concurrently, or the blocks may sometimes be performed in the reverse order, depending upon the functionality involved. Also, other blocks may be added in addition to the illustrated blocks in a flowchart or block diagram. Some blocks may be optional. For example, operation 506 through operation 532 may be optional. As another example, operation 610 through operation 624 may be optional. Additionally, operation 710 through operation 714 may be optional.

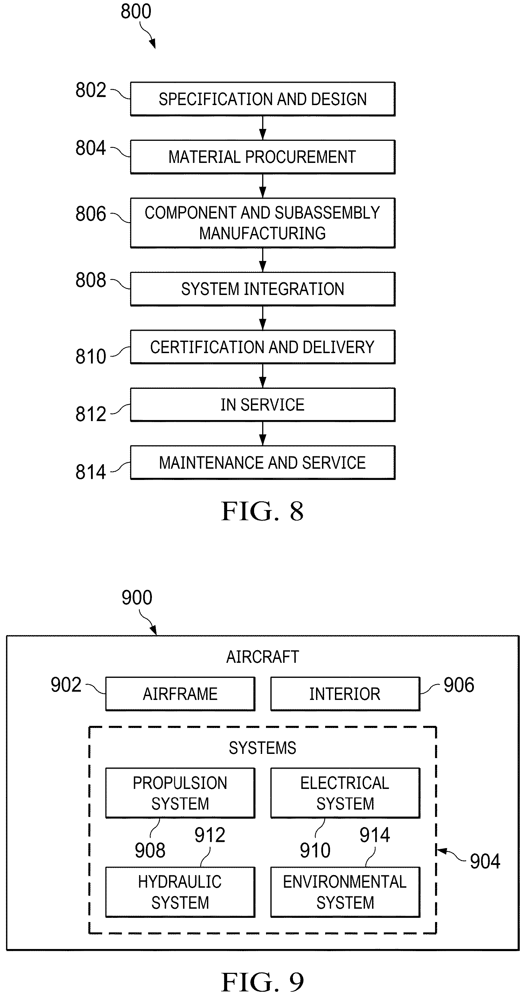

Illustrative examples of the present disclosure may be described in the context of aircraft manufacturing and service method 800 as shown in Figure 8 and aircraft 900 as shown in Figure 9. Turning first to Figure 8, an illustration of an aircraft manufacturing and service method in a form of a block diagram is depicted in accordance with an illustrative example. During pre-production, aircraft manufacturing and service method 800 may include specification and design 802 of aircraft 900 in Figure 9 and material procurement 804.

During production, component and subassembly manufacturing 806 and system integration 808 of aircraft 900 takes place. Thereafter, aircraft 900 may go through certification and delivery 810 in order to be placed in service 812. While in service 812 by a customer, aircraft 900 is scheduled for routine maintenance and service 814, which may include modification, reconfiguration, refurbishment, or other maintenance and service.

Each of the processes of aircraft manufacturing and service method 800 may be performed or carried out by a system integrator, a third party, and/or an operator. In these and certain other examples, the operator may be a customer. For the purposes of this description, a system integrator may include, without limitation, any number of aircraft manufacturers and major-system subcontractors; a third party may include, without limitation, any number of vendors, subcontractors, and suppliers; and an operator may be an airline, a leasing company, a military entity, a service organization, and so on.

With reference now to Figure 9, an illustration of an aircraft in a form of a block diagram is depicted in which an illustrative example may be implemented. In this and certain other examples, aircraft 900 is produced by aircraft manufacturing and service method 800 of Figure 8 and may include airframe 902 with plurality of systems 904 and interior 906. Examples of systems 904 include one or more of propulsion system 908, electrical system 910, hydraulic system 912, and environmental system 914. Any number of other systems may be included.

Apparatuses and methods embodied herein may be employed during at least one of the stages of aircraft manufacturing and service method 800. One or more illustrative examples may be manufactured or used during at least one of component and subassembly manufacturing 806, system integration 808, in service 812, or maintenance and service 814 of Figure 8.

To qualify and use lamination automated measurement systems in production, the measurement systems are tested to understand the limits and behaviors of their "deadbands" when measuring overlaps and gaps. At least some illustrative examples lower the cost and time needed to determine this behavior when compared to using multiple discrete layups for the same purpose. In some illustrative examples, the taper layup and methods presented produce a more detailed analysis of the behavior of the measurement equipment within the deadband. In some illustrative examples, the taper layup methods produce a more accurately identified deadband for the measurement equipment.

The description of the different illustrative examples has been presented for purposes of illustration and description, and is not intended to be exhaustive or limited to the examples in the form disclosed. Many modifications and variations will be apparent to those of ordinary skill in the art. Further, different illustrative examples may provide different features as compared to other illustrative examples. The example or examples selected are chosen and described in order to best explain the principles of the examples, the practical application, and to enable others of ordinary skill in the art to understand the disclosure for various example with various modifications as are suited to the particular use contemplated.

Further examples are provided in the clauses below.
1. A method (600) of selecting measurement equipment (202) comprising:
   repeatedly (602) measuring a layup (218, 302) of adjacent strips (222) of material along a material interface (238) by measurement equipment (202) to form measurement data (248), the material interface (238) comprising a varying overlap (234, 310) and a varying gap (236, 312) forming a taper interface (240);
   identifying (604) a minimum overlap (208) measurable to a desired reliability (212) by the measurement equipment (202) from the measurement data (248);
   identifying (606) a minimum gap (210) measurable to the desired reliability (212) by the measurement equipment (202) from the measurement data (248); and
   defining (608) a deadband (204) for the measurement equipment (202) between the minimum overlap (208) and the minimum gap (210).
2. The method (600) of clause 1 further comprising:
   comparing (620) the deadband (204) of the measurement equipment (202) to a tolerance range (258) of a composite layup (218, 302); and
   selecting (622) the measurement equipment (202) for inspection of the composite layup (218, 302) if the deadband (204) of the measurement equipment (202) is contained within the tolerance range (258) for the composite layup (218, 302).
3. The method (600) of clause 2, wherein (624) the tolerance range (258) is bounded by an overlap tolerance (262) and a gap tolerance (260) of the composite layup (218, 302).
4. The method (600) of any of clauses 1 to 3, wherein (610) defining the deadband (204) comprises identifying differences (252) between measured values (250) and true values (246) that are outside of a measurement tolerance and including the true values (246) associated with differences (252) outside of the measurement tolerance in the deadband (204).
5. The method (600) of any of clauses 1 to 4, wherein defining the deadband (204) comprises:
   identifying (612 true values (246) that correlate with measured values (250) that are zero in at least fifty percent of the measurements to form identified values; and
   wherein (614) defining the deadband (204) comprises including the identified values in the deadband (204).
6. The method (600) of any of clauses 1 to 5, further comprising:
   identifying (616) true values (246) that correlate with measured values (250) that are zero in at least fifty percent of the measurements to form identified values; and
   wherein (618) defining the deadband (204) comprises including the identified values in the deadband (204).
7. A method (700) of selecting measurement equipment (202) comprising:
   laying up (702) a second strip (228, 306) of material adjacent a first strip (224, 304) of material to form a taper interface (240) with a varying gap (236, 312) and a varying overlap (234, 310);
   repeatedly (704) measuring the taper interface (240) by measurement equipment (202) to form measurement data (248);
   identifying (706) a deadband (204) for the measurement equipment (202) between a minimum overlap (208) measurable to a desired reliability (212) from the measurement data (248) and a minimum gap (210) measurable to the desired reliability (212) from the measurement data (248); and
   selecting (708) the measurement equipment (202) for inspection of a composite layup (218, 302) if the deadband (204) of the measurement equipment (202) is contained within a tolerance range (258) for a composite layup (218, 302).
8. The method (700) of clause 7, wherein (714) the tolerance range (258) is bounded by an overlap tolerance (262) and a gap tolerance (260) of the composite layup (218, 302).
9. The method (700) of clause 7 or 8, further comprising:
   identifying (710) true values (246) that correlate with measured values (250) that are zero in at least fifty percent of the measurements to form identified values; and
   wherein (712) identifying the deadband (204) comprises including the identified values in the deadband (204).

## Claims

1. A method (500) of selecting measurement equipment (202) comprising:
repeatedly (502) measuring a layup (218, 302) of adjacent strips (222) of material along a material interface (238) by measurement equipment (202) to form measurement data (248); and
identifying (504) a deadband (204) of gaps and overlaps for the measurement equipment (202) within the measurement data (248).

2. The method (500) of claim 1, wherein identifying the deadband (204) comprises:
identifying (516) a minimum overlap (208) measurable to a desired reliability (212) by the measurement equipment (202); and
identifying (518) a minimum gap (210) measurable to the desired reliability (212) by the measurement equipment (202).

3. The method (500) of claim 1 or 2, further comprising:
evaluating (520) measurement data (248) within the deadband (204) to identify unexpected values in the measurement data (248) within the deadband (204).

4. The method (500) of any preceding claim, further comprising:
laying up (506) a second strip (228, 306) of material adjacent a first strip (224, 304) of material to form a taper interface (240) with a varying gap (236, 312) and a varying overlap (234, 310) to form the layup (218, 302) of adjacent strips (222) of material.

5. The method (500) of any preceding claim, further comprising:
laying up (508) a first strip (224, 304) of material at a first angle (226); and
laying up (510) a second strip (228, 306) of material at a second angle (230) over the first strip (224, 304) of material, wherein the first angle (226) and the second angle (230) are different.

6. The method (500) of claim 5, wherein (512) laying up the second strip (228, 306) of material at the second angle (230) lays up the second strip (228, 306) of material such that there is a varying gap (236, 312) between the first strip (224, 304) and the second strip (228, 306) and a varying overlap (234, 310) of the second strip (228, 306) on the first strip (224, 304).

7. The method (500) of any preceding claim, wherein (522) identifying the deadband (204) comprises identifying differences (252) between measured values (250) and true values (246) that are outside of a measurement tolerance and including the true values (246) associated with differences (252) outside of the measurement tolerance in the deadband (204).

8. The method (500) of any preceding claim, wherein identifying the deadband (204) comprises:
identifying (524) true values (246) that correlate with measured values (250) that are zero (254) in at least fifty percent of the measurements to form identified values; and
wherein (526) identifying the deadband (204) comprises including the identified values in the deadband (204).

9. The method (500) of any preceding claim, wherein the layup (218, 302) comprises composite material (220).

10. The method (500) of claim 9, wherein the composite material (220) is a pre-impregnated or dry fiber composite.

11. The method (500) of claim 10, wherein (514) the adjacent strips (222) of material comprise at least one of a thermoplastic prepreg material, a thermoset prepreg material, or a dry fiber material.

12. The method of any of claims 10 or 11, wherein the composite material (220) is a unidirectional carbon fiber tape pre-impregnated with an epoxy resin.

13. The method (500) of any preceding claim, further comprising:
comparing (528) the deadband (204) of the measurement equipment (202) to a tolerance range (258) of a composite layup (218, 302); and
approving (530) the measurement equipment (202) for inspection of the composite layup (218, 302) if the deadband (204) of the measurement equipment (202) is contained within the tolerance range (258) for the composite layup (218, 302).

14. The method (500) of claim 13, wherein (532) the tolerance range (258) is bounded by an overlap tolerance (262) and a gap tolerance (260) of the composite layup (218, 302).

15. The method of any preceding claim, further comprising forming an aircraft component from the layup (218, 302) and, optionally, wherein the aircraft component is a composite wing skin.
